(19) Europäisches Patentamt
European Patent Office
Office européen des brevets

(11) **EP 3 890 596 B1**

(12) **EUROPEAN PATENT SPECIFICATION**

(45) Date of publication and mention
of the grant of the patent:
**05.06.2024 Bulletin 2024/23**

(21) Application number: **20749161.4**

(22) Date of filing: **30.01.2020**

(51) International Patent Classification (IPC):
**A61B 5/00** *(2006.01)*     **A61B 5/11** *(2006.01)*
**A61B 5/024** *(2006.01)*

(52) Cooperative Patent Classification (CPC):
**A61B 5/02405; A61B 5/0022; A61B 5/02416;
A61B 5/1118; A61B 5/1172; A61B 5/4812;
A61B 5/4815; A61B 5/681; A61B 5/7278;
A61B 5/7455;** A61B 5/01; A61B 2562/0204;
A61B 2562/0219; A61B 2562/0247; A61B 2562/029

(86) International application number:
**PCT/KR2020/001436**

(87) International publication number:
**WO 2020/159259 (06.08.2020 Gazette 2020/32)**

(54) **METHOD FOR CALCULATING RECOVERY INDEX BASED ON REM SLEEP STAGE AND ELECTRONIC DEVICE THEREOF**

VERFAHREN ZUR BERECHNUNG DES ERHOLUNGSINDEXES AUF BASIS DER REM-SCHLAFPHASE UND ELEKTRONISCHE VORRICHTUNG DAFÜR

PROCÉDÉ DE CALCUL D'INDICE DE RÉCUPÉRATION SUR LA BASE D'UN STADE DE SOMMEIL PARADOXAL ET DISPOSITIF ÉLECTRONIQUE ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2019 KR 20190011711**

(43) Date of publication of application:
**13.10.2021 Bulletin 2021/41**

(73) Proprietor: **Samsung Electronics Co., Ltd.
Suwon-si, Gyeonggi-do 16677 (KR)**

(72) Inventors:
• **LEE, Donghyun
Suwon-si, Gyeonggi-do 16677 (KR)**

• **LEE, Wonkyu
Suwon-si, Gyeonggi-do 16677 (KR)**
• **NAM, Sangbeom
Suwon-si, Gyeonggi-do 16677 (KR)**

(74) Representative: **Gulde & Partner
Patent- und Rechtsanwaltskanzlei mbB
Wallstraße 58/59
10179 Berlin (DE)**

(56) References cited:
**EP-A1- 2 842 491     JP-A- 2006 271 897
JP-A- 2016 218 563     JP-A- 2018 126 422
KR-A- 20170 142 227     US-A1- 2012 232 414
US-A1- 2015 265 212**

EP 3 890 596 B1

**Description**

**Technical Field**

One or more embodiments disclosed herein generally relate to a method for calculating a recovery index based on REM sleep stage and an electronic device thereof.

**Background Art**

Recently, technologies for monitoring biosignals of a user of electronic devices such as smartphones and wearable devices have been developed. It may be possible to monitor the physical condition and the sleep state of the user using various sensing technologies. Methods and devices for monitoring biosignals of a user are disclosed e.g. in EP 2 842 491 A1 and US 2015/265212 A1.

People are stressed when harmful stimulation is applied to their bodies, and such stress may refer to mental and physical fatigue in the user's body. Such stress can be alleviated through sleep.

Sleep states include Non-Rapid Eye Movement (NREM) sleep state and Rapid Eye Movement (REM) sleep state. It has been known that growth, better immunity against diseases, and recovery of neural, skeletal, and muscular systems can be achieved via sleep. However, the mechanisms of sleep are not yet exactly known, and are being actively studied. Whether sleep influences recovery from stress is also the subject of active study, and there are reports indicating that REM sleep time in particular can alleviate stress.

The above information is presented as background information only to assist with an understanding of the disclosure. No determination has been made, and no assertion is made, as to whether any of the above might be applicable as prior art with regard to the disclosure.

**Disclosure of Invention**

**Solution to Problem**

Several methods of determining sleep quality are known in the art. These methods may be based on the sleep state of the user. In particular, one such method may be based on the period of wakefulness during sleep and the ratios of the lengths of various sleep stages to the entire length of the sleep period. However, such methods of determining sleep quality does not have any way to infer how the sleep helps the user to recover from stress. Accordingly, methods of addressing recovery from stress has not been proposed.

The technical subjects pursued in the disclosure may not be limited to the above mentioned technical subjects, and other technical subjects which are not mentioned may be clearly understood, through the following descriptions, by those skilled in the art of the disclosure.

According to the invention, an electronic device according to claim 1 is provided. The electronic device includes: a communication module configured to perform communication with an external device; a motion sensor configured to sense movement of the electronic device; a biosensor configured to obtain biological information of a user of the electronic device; at least one processor operationally connected with the communication module, the motion sensor, and the biosensor; and at least one memory operationally connected with the at least one processor. The at least one memory stores instructions that, when executed by the at least one processor, cause the at least one processor to: obtain Rapid Eye Movement (REM) sleep time periods and stress data before and after sleep, based on first data obtained by the motion sensor and second data obtained by the biosensor, set parameters for calculating a recovery index, based on the obtained REM sleep time periods and the stress data before and after sleep, and calculate the recovery index, based on the set parameters.

According to an embodiment, which is useful for the understanding of but not part of the invention as claimed, a system includes: a sensing device; and a processing device. The sensing device includes a motion sensor configured to sense movement of a user, a biosensor configured to obtain biological information of the user, and a first communication module configured to perform communication with the processing device. The processing device includes a second communication module configured to perform communication with the sensing device, at least one processor operationally connected with the second communication module, and at least one memory operationally connected with the at least one processor. The sensing device and the processing device are implemented as different pieces of hardware. The sensing device transmits first data obtained by the motion sensor and second data obtained by the biosensor to the processing device through the communication module. The at least one memory of the processing device stores instructions that, when executed by the at least one processor, cause the at least one processor to: obtain Rapid Eye Movement (REM) sleep time periods and stress data before and after sleep, based on first data and second data received from the sensing device, set parameters for calculating a recovery index, based on the obtained REM sleep time periods and/or the stress data before and after sleep, and calculate the recovery index, based on the set parameters.

According to the invention, a method of operating an electronic device according to claim 8 is provided. The method includes: obtaining first data including information about movement of a user; obtaining second data including biological information of the user; obtaining Rapid Eye Movement (REM) sleep time periods and stress data before and after sleep, based on the first data and the second data; setting parameters for calculating a recovery index, based on the obtained REM sleep time period and stress data before and after sleep; and calculating the recovery index, based on the set parameters.

Additional aspects will be set forth in part in the description which follows and, in part, will be apparent from the description, or may be learned by practice of the presented embodiments.

## Brief Description of Drawings

The above and other aspects, features, and advantages of certain embodiments of the disclosure will be more apparent from the following description taken in conjunction with the accompanying drawings, in which:

FIG. 1 is a block diagram illustrating an electronic device in a network environment according to various embodiments.
FIG. 2A is a perspective view of a front surface of a mobile electronic device according to an embodiment.
FIG. 2B is a perspective view of a rear surface of the electronic device of FIG. 2A.
FIG. 2C is a perspective view of the electronic device of FIG. 2A.
FIG. 3 is a block diagram illustrating a sensing device that can be attached to a living body;
FIG. 4 is graphs illustrating a comparison between REM sleep pattern estimated based on Heart Rate (HR) and/or Heart Rate Variation (HRV), and sleep pattern measured by polysomnography;
FIG. 5 is graphs illustrating sleep pattern estimated, based on HR, HRV, and/or a movement parameter and a sleep pattern measured by polysomnography in accordance with an embodiment;
FIG. 6 is a block diagram illustrating an example of the functional configuration of an electronic device according to an embodiment;
FIG. 7 is a flowchart showing the operation in which an electronic device according to an embodiment calculates a recovery index; and
FIG. 8 is a flowchart showing the operation in which an electronic device according to an embodiment obtains REM sleep time and stress data.

In the description of drawings, the same or similar components may be given the same or similar reference numerals.

## Best Mode for Carrying out the Invention

Hereafter, various embodiments are described in detail with reference to the accompanying drawings.

One or more embodiments disclosed herein relate to a system that can calculate and provide a recovery index by calculating mental stress before and after sleep, based on data measured by sensors, and by determining how the user's body recovers based on the Rapid Eye Movement (REM) stage of sleep.

A method and an electronic device according to an embodiment relate to a system that may be able to ascertain the degree of recovery from stress through REM sleep by comparing a stress value measured before the user falls asleep and a stress value measure after the user falls asleep.

A method and an electronic device according to an embodiment relate to a system that may be able to help the user recover from stress by informing the user of the necessary REM sleep time or providing various services for recovery to the user when the user is not sufficiently recovered.

A method and an electronic device according to an embodiment relate to a system that may be able to determine the quality of sleep and inform the same to the user by comparing the total amount of stress alleviated through sleep, where the total amount of stress may be measured for a predetermined time period.

A method and an electronic device according to an embodiment relate to a system that may be able to prevent chronic stress of the user by providing feedback, to the user, on the extent the acute stress of the user. The extent of the stress may refer the continuous length of time in which the stress is exhibited and/or how often the user exhibits stress.

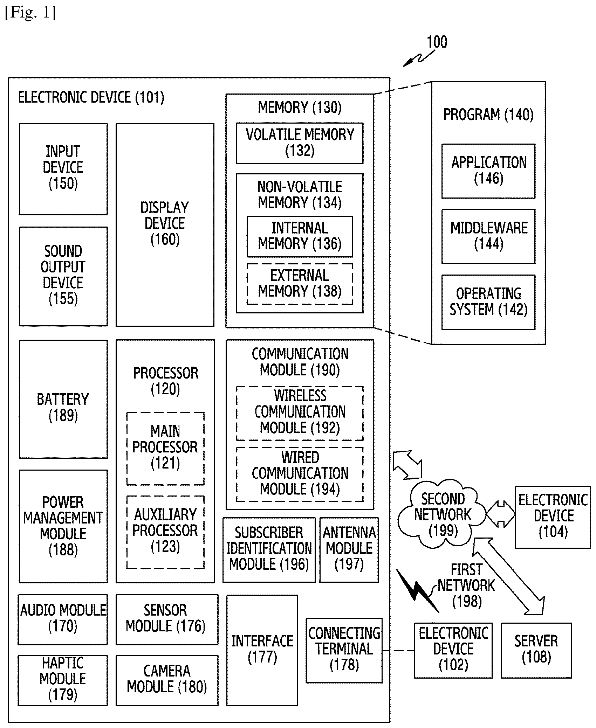

FIG. 1 is a block diagram illustrating an electronic device 101 in a network environment 100 according to an embodiment. Referring to FIG. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input device 150, a sound output device 155, a display device 160, an audio module 170, a sensor module 176, an interface 177, a haptic module 179, a camera module 180, a power management module 188, a battery 189, a communication module 190, a subscriber identification module (SIM) 196, or an antenna module 197. In some embodiments, at least one (e.g., the display device 160 or the camera module 180) of the components may be omitted from the electronic device 101, or one or more other components may be added in the electronic device 101. In some embodiments, some of the components may be implemented as single integrated circuitry. For example, the sensor module 176 (e.g., a fingerprint sensor, an iris sensor, or an illuminance sensor) may be implemented as embedded in the display device 160 (e.g., a display).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may load a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and an auxiliary processor 123 (e.g., a graphics processing unit (GPU), an image signal processor (ISP), a sensor hub processor, or a communication processor (CP)) that is operable independently from, or in conjunction with, the main processor 121. Additionally or alternatively, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display device 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input device 150 may receive a command or data to be used by other component (e.g., the processor 120) of the electronic device 101, from the outside (e.g., a user) of the electronic device 101. The input device 150 may include, for example, a microphone, a mouse, a keyboard, or a digital pen (e.g., a stylus pen).

The sound output device 155 may output sound signals to the outside of the electronic device 101. The sound output device 155 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display device 160 may visually provide information to the outside (e.g., a user) of the electronic device 101. The display device 160 may include, for example, a display, a hologram device, or a projector and control circuitry to control a corresponding one of the display, hologram device, and projector. According to an embodiment, the display device 160 may include touch circuitry adapted to detect a touch, or sensor circuitry (e.g., a pressure sensor) adapted to measure the intensity of force incurred by the touch.

The audio module 170 may convert a sound into an electrical signal and vice versa. According to an embodiment, the audio module 170 may obtain the sound via the input device 150, or output the sound via the sound output device 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as BluetoothTM, wireless-fidelity (Wi-Fi) direct, or infrared data association (IrDA)) or the second network 199 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The antenna module 197 may transmit or receive a signal or power to or from the outside (e.g., the external electronic device) of the electronic device 101. According to an embodiment, the antenna module 197 may include a plurality of antennas. In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 and 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, or client-server computing technology may be used, for example.

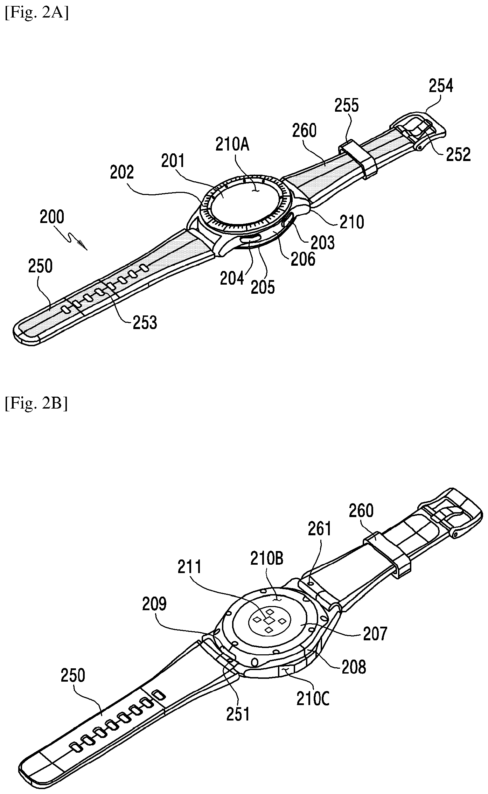

FIG. 2A is a perspective view of a front surface of a mobile electronic device 200 according to an embodiment. FIG. 2B is a perspective view of a rear surface of the electronic device 200 of FIG. 2A. The electronic device 200 shown in FIG. 2A and FIG. 2B may correspond to the electronic device 101 shown in FIG. 1.

Referring to FIGs. 2A and 2B, the electronic device 200 according to an embodiment may include a housing including a first surface (or a front surface) 210A, a second surface (or a rear surface) 210B, and a side surface 210C surrounding a space between the first surface 210A and the second surface 210B. Fastening members 250 and 260 may be connected to at least portions of the housing 210 and the fastening members 250 and 260 are configured to detachably fasten the electronic device 200 to a portion (e.g., wrist or ankle) of the body of the user. In another embodiment (not illustrated), the housing may refer to a structure that forms some of the first surface 210A, the second surface 210B, and the side surface 210C of FIG. 2A. According to an embodiment, the first surface 210A may be formed by a front plate 201 of FIG. 2A (e. g., a glass plate including at least one coating layer or a polymer plate), at least a portion of which is substantially transparent. The second surface 210B may be formed by a rear plate 207 that is substantially opaque. The rear plate 207, for example, may be made of coated or colored glass, ceramics, polymer, metal (e. g., aluminum, stainless steel (STS), or magnesium), or a combination of at least two thereof. The side surface 210C is coupled to the front plate 201 and the rear plate 207, and may be formed by a side bezel structure 206 (or "a side member") including metal and / or polymer. In some embodiments, the rear plate 207 and the side bezel structure 206 may be integrated and may be made of the same materials (e. g., metallic material such as aluminum). The fastening members 250 and 260 may be formed of various material and have various shapes. A single body or a plurality of unit links that may move with respect to each other may be made of woven fabric, leather, rubber, urethane, metal, ceramics, or a combination of at least two thereof.

According to an embodiment, the electronic device 200 may include at least one of a display 220, audio modules 205 and 208, a sensor module 211, key input devices 202, 203, and 204 and a connector hole 209. In some embodiments, at least one (e. g., the key input devices 202, 203, and 204, the connector hole 209, or the sensor module 211) may be omitted from the electronic device 200 or another component may be additionally included in the electronic device 200. In another embodiment, a sensor module 211 may be a part of the sensor module 176 shown in FIG. 1.

The display 220, for example, may be exposed through the front plate 201. The shape of the display 220 may correspond to the shape of the front plate 201, which may be various shapes, such as circular, elliptical, or polygonal. The display 220 may be coupled to or be disposed to be adjacent to a touch detection circuit, a pressure sensor that may measure the strength (a pressure) of a touch, and / or a fingerprint sensor.

The audio modules 205 and 208 may include microphone hole 205 and speaker hole 208. A microphone for obtaining external sound may be disposed in the interior of the microphone hole 205. In some embodiments, a plurality of microphones and a plurality of microphone holes may be disposed to detect the direction of the sound. The speaker hole 208 may be used by a speaker and/or a communication receiver to output sound. In some embodiments, the speaker hole 208 and the microphone hole 205 may be combined into a single hole or the speaker hole 208 may be omitted because the speaker does not require a corresponding hole (e. g., a piezoelectric speaker).

The sensor module 211 may generate electrical signal or data value corresponding to operation states of the electronic device 200 or a condition outside the electronic device 200. The sensor module 211, for example, may include the biosensor module 211 (e.g., heartbeat sensor or electrode sensor) disposed on the second surface 210B of the housing 210.

The electronic device 200 may further include another sensor module (not illustrated), such as a gesture sensor, a gyro sensor, an atmospheric pressure sensor, a magnetic sensor, an acceleration sensor, a grip sensor, a color sensor, an infrared (IR) sensor, a biometric sensor, a temperature sensor, a humidity sensor, or an illumination sensor.

The key input devices 202, 203, and 204 may include a wheel key 202 disposed on the first surface 210A of the housing 210 and rotatable in at least one direction. The key input devices 202, 203, and 204 may further include side key buttons 203 and 204 disposed on the side surface 210C of the housing 210. The wheel key 202 may have a shape corresponding to the shape of the front plate 201. In another embodiment, one or more hardware key input devices 202, 203, and 204 may be omitted, and the omitted key input device may be implemented in software and be displayed on the display 220.

The connector hole 209 may accommodate a connector (e. g., USB connector) for transmitting and receiving power and / or data to and from an external electronic device, and may include another connector hole (not illustrated) that may accommodate a connector for transmitting and receiving audio signal to and from an external electronic device. The electronic device 200, for example, may further include a connector cover (not illustrated) configured to cover at least a portion of the connector hole 209 to prevent external foreign substances from entering the connector hole 209.

The fastening members 250 and 260 may be detachably fastened to at least a partial area of the housing 210 by using locking members 251 and 261. The fastening members 250 and 260 may include one or more of a fixing member 252, a fixing member coupling hole 253, a band guide member 254, and a band fixing ring 255.

The fixing member 252 may be configured to fix the housing 210 and the fastening members 250 and 260 to a portion (e. g., wrist or ankle) of the body of the user. The fixing member coupling hole 253 may fix the housing 210 and the fastening members 250 and 260 to a portion of the body of the user in correspondence to the fixing member 252. The band guide member 254 may be configured to restrict the motion range of the fixing member 252 when the fixing member 252 is coupled to the fixing member coupling hole 253 so that the fastening members 250 and 260 are fastened to be attached to the portion of the body of the user. The band fixing ring 255 may restrict motion ranges of the fastening members 250 and 260 when the fixing member 252 and the fixing member coupling hole 253 are coupled to each other.

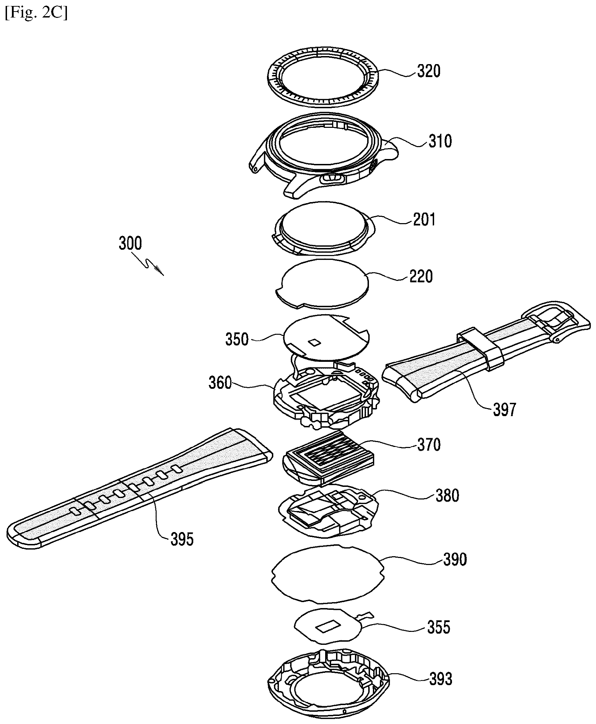

FIG. 2C is a perspective view of the electronic device of FIG. 2A.

Referring to FIG. 2C, the electronic device 300 may include a side bezel structure 310, a wheel key 320, a front plate 201, a display 220, a first antenna 350, a second antenna 355, a support member 360 (e. g., a bracket), a battery 370, a printed circuit board 380, a sealing member 390, a rear plate 393 and fastening members 395 and 397. Some of the components of the electronic device 300 may be the same as or similar to corresponding components of the electronic device 200 of FIGs. 2A and 2B, and duplicative descriptions thereof will be omitted. The support member 360 may be disposed in the interior of the electronic device 300 to be connected to the side bezel structure 310 or to be integrally formed with the side bezel structure 310. The support member 360, for example, may be made of metallic material and / or nonmetallic material (e. g., polymer). The display 220 may be coupled to one surface of the support member 360, and the printed circuit board 380 may be coupled to the opposite surface of the support member 360. A processor, a memory, and / or an interface may be mounted on the printed circuit board 380. The processor, for example, may include one or more of a central processing unit, an application processor, a graphic processing unit (GPU), an application processor, a sensor processor, or a communication processor. The processor may include a microprocessor or any suitable type of processing circuitry, such as one or more general-purpose processors (e.g., ARM-based processors), a Digital Signal Processor (DSP), a Programmable Logic Device (PLD), an Application-Specific Integrated Circuit (ASIC), a Field-Programmable Gate Array (FPGA), a Graphical Processing Unit (GPU), a video card controller, etc. In addition, it would be recognized that when a general purpose computer accesses code for implementing the processing shown herein, the execution of the code transforms the general purpose computer into a special purpose computer for executing the processing shown herein. Certain of the functions and steps provided in the Figures may be implemented in hardware, software or a combination of both and may be performed in whole or in part within the programmed instructions of a computer. No claim element herein is to be construed under the provisions of 35 U.S.C. 112(f), unless the element is expressly recited using the phrase "means for." In addition, an artisan understands and appreciates that a "processor" or "microprocessor" may be hardware in the claimed disclosure. Under the broadest reasonable interpretation, the appended claims are statutory subject matter in compliance with 35 U.S.C. §101.

The memory, for example, may include volatile and / or nonvolatile memory. The interface, for example, may implement High Definition Multimedia Interface (HDMI), Universal Serial Bus (USB), SD card interface, and / or an audio interface. The interface, for example, may electrically or physically connect the electronic device 300 to an external electronic device, and may include a USB connector, an SD card / MMC connector, and an audio connector. In certain embodiments, the memory can store a previously recorded fingerprint for matching by the finger print sensor.

The battery 370 is a device for supplying electric power to at least one component of the electronic device 300, and for example, may include a primary battery that cannot be recharged, a secondary battery that may be recharged, or a fuel cell. At least a portion of the battery 370, for example, may be parallel to the printed circuit board 380. The battery 370 may be integrally disposed in the interior of the electronic device 300, or may be disposed to be detachable from the electronic device 300.

The first antenna 350 may be disposed between the display 220 and the support member 360. The first antenna 350, for example, may include a near field communication (NFC) antenna, a wireless charging antenna, and / or a magnetic secure transmission (MST) antenna. The first antenna 350, for example, may perform short-range communication with an external device, may wirelessly transmit and receive electric power for charging the battery 370, and may transmit short-range communication signal or magnetism-based signal including payment data. In another embodiment, an antenna structure of the first antenna 350 may be formed by one or a combination of the side bezel structure 310 and / or the support member 360.

The second antenna 355 may be disposed between the printed circuit board 380 and the rear plate 393. The second antenna 355, for example, may include a near field communication (NFC) antenna, a wireless charging antenna, and / or a magnetic secure transmission (MST) antenna. The second antenna 355, for example, may perform short-range communication with an external device, may wirelessly transmit and receive electric power for charging the battery 370, and may transmit short-range communication signal or magnetism-based signal including payment data. In another embodiment, an antenna structure of the second antenna 355 may be formed by one or a combination of the side bezel structure 310 and / or the rear plate 393.

The sealing member 390 may be located between the side bezel structure 310 and the rear plate 393. The sealing member 390 may be configured to prevent moisture and foreign substances from entering the space surrounded by the side bezel structure 310 and the rear plate 393 from the outside.

According to certain embodiments, some of the components shown in FIG. 2A, FIG. 2B, and FIG. 2C ma correspond to some of the components shown in FIG. 1. For example, the display 220 may correspond to the display device 160, the audio modules 205 and 208 may correspond to the audio module 170, the sensor module 211 may correspond to the sensor module 176, the key input devices 202, 203, and 204 may correspond to the input device 150, and the battery 370 may correspond to the battery 189.

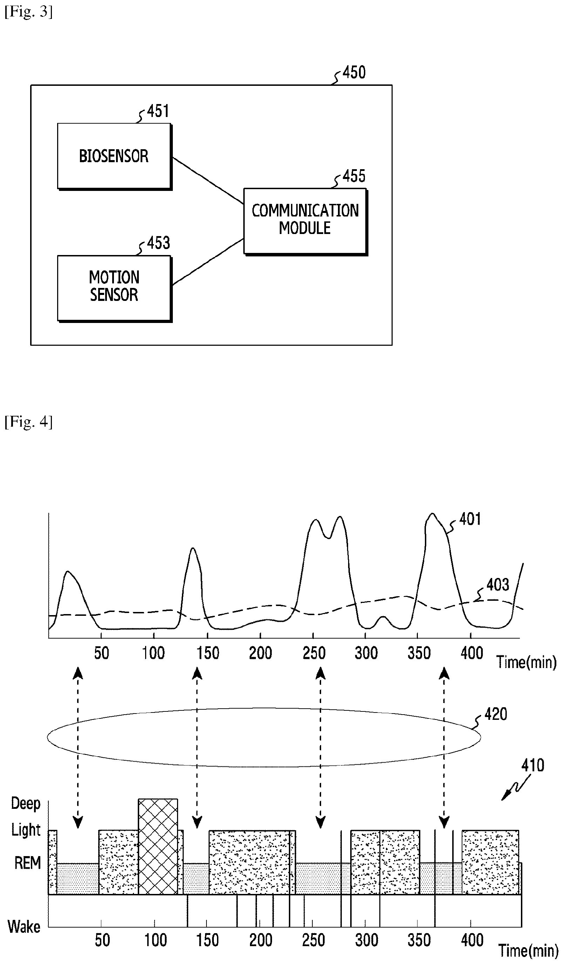

FIG. 3 is a block diagram illustrating a sensing device 450 that can be attached to a living body.

Referring to FIG. 3, the sensing device 450 may include a biosensor 451 that can be attached to the body of the user. The biosensor may be a patch or band type sensor that can measure biological information. Specifically, patch sleep sensors may include patches that are adhered to, for example, the head of the user. Band type sleep sensors may include a headband that is placed on the user's head. The sensing device 450 may further include a motion sensor 453 that can measure movement of the body, and a communication module 455 that transmits measured biological information data to another device. The sensing device 450 may further include a processor (not shown) for controlling and directing the processing of data. The communication module 455 can perform communication with a separate processing device (e.g., the electronic device 101 shown in FIG. 1 or a smartphone) and can transmit biological information data measured by the biosensor 451 and movement information data measured by the motion sensor 453 to the processing device via various data communication protocols, such as wireless protocols (e.g. Bluetooth, BLE, WiFi, ZigBee communication protocols), as well as wired protocols (e.g. Ethernet and Universal Serial Bus (USB)).

Compared to sensors in the watch shown in FIG. 2A and FIG. 2B, the patch or band type sensing device 450 may obtain more extensive and precise sleep data because they include a plurality of sensing devices that are attached to various places on the user's body.

There are various theories about why humans sleep. In one theory, sleep is required for restoration and recovery. According to this theory, sleep may be necessary for recovering the physiological process that keeps body and mind healthy and enables them to function well. Under this theory, Non-Rapid Eye Movement (NREM) sleep stage may be important for physiological function recovery and Rapid Eye Movement (REM) sleep stage may be important for mental function recovery.

Various studies support the theory of restoration and recovery through sleep and the instant disclosure operates under this theory. The instant disclosure provides a relationship between REM sleep time and a recovery index of stress by addressing the mental function recovery of REM sleep stage.

FIG. 4 is graphs illustrating a comparison between REM sleep pattern 401 estimated based on Heart Rate (HR) and/or Heart Rate Variation (HRV), and sleep pattern 410 measured by polysomnography.

Referring to FIG. 4, the electronic device 101 can set a critical value 403 and can estimate the period in which REM sleep pattern 401 occurs. This is done by determining whether the HR and/or HRV is larger than the set critical value. If so, the period in which the HR and/or HRV is larger than the set critical value is determined to be the REM sleep period. By comparing the REM sleep period estimated based on HR and/or HRV with the REM sleep period detected based on polysomnography (420), it is possible to find out that there is a relationship between them. That is, it can be seen that the REM sleep period estimated based on the HR and/or HRV corresponds to the actual REM sleep period of the user. Accordingly, the electronic device 101 can obtain HR and/or HRV values and can estimate the REM sleep period based on those values.

Further, the electronic device 101 can distinguish shallow sleep from deep sleep using HR and/or HRV. Sleep can be classified into NREM sleep and REM sleep. The NREM sleep can be classified into three kinds of sleep stages N1, N2, and N3 in accordance with the depth, in which N1 and N2 can be classified into a shallow sleep and N3 can be classified into deep sleep or slow wave sleep.

According to an embodiment, the electronic device 101 can distinguish shallow sleep, deep sleep, and REM sleep periods, using at least one of HR, HRV, or a movement parameter (actigraphy).

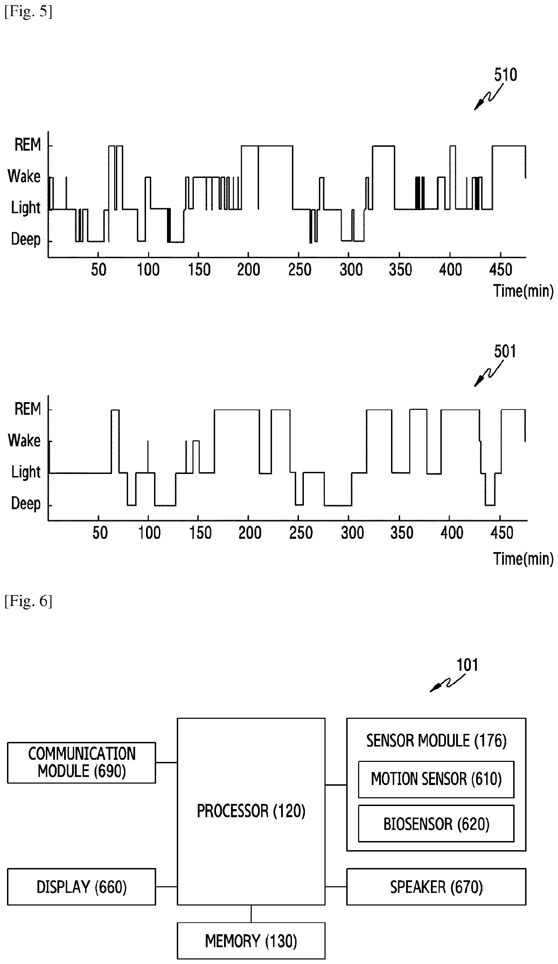

FIG. 5 is graphs illustrating a sleep pattern 501 estimated based on HR, HRV, and/or a movement parameter and a sleep pattern 510 measured by polysomnography in accordance with an embodiment.

Referring to FIG. 5, it can be seen that there is a meaningful relationship between the sleep pattern 501 estimated based on HR, HRV, and/or the movement parameter, and the sleep pattern 510 measured by polysomnography. Accordingly, the electronic device 101 can obtain HR, HRV, and/or the movement parameter, using the sensor module 211, and can estimate the sleep pattern of the user, based on the obtained HR, HRV, and/or movement parameter.

FIG. 6 is a block diagram illustrating an example of the functional configuration of the electronic device 101 according to an embodiment. The electronic device 101 may be a wearable device (e.g., smartwatch). The electronic device 101, for example, may correspond to the electronic device 200 shown in FIG. 2A.

Referring to FIG. 6, the electronic device 101 may include a sensor module 176, a communication module 690, a display 660, a speaker 670, a processor 120, and/or a memory 130. The disclosure is not limited thereto, and at least one of the components may be omitted or one or more other components may be added in the electronic device 101.

The sensor module 176 may include a motion sensor 610 and a biosensor 620. The biosensor 620 may correspond to the sensor module 211 shown in FIG. 2A.

The motion sensor 610 can sense (or obtain) various kinds of signals or data related to the activity state of the user of the electronic device 101. The motion sensor 610, for example, may include at least one of an acceleration sensor (e.g., an accelerometer), a gyro sensor (e.g., gyroscope), or a geomagnetic sensor. Data obtained through at least one of the acceleration sensor, the gyro sensor, or the geomagnetic sensor can be used to detect the activity state of the user who is wearing or carries the electronic device 101. The method of discriminate the activity states of the user may be various. For example, the activity states of the user may include an active state, a static state (e.g., a static stable state or a static unstable state), and a stable state.

According to an embodiment, the motion sensor 610 can detect the direction of gravity acting on the electronic device 101 through the acceleration sensor. The motion sensor 610 can detect movement or movement path of the electronic device 101 through the gyro sensor. The electronic device 101 can discriminate the activity states of the user using the results detected using the motion sensor 610. The electronic device 101 can estimate the wake-up time of the user based on the activity states.

The biosensor 620 can sense or obtain biosignals of the user who is wearing the electronic device 101. The biosensor 620, for example, may include a photoplethysmogram (PPG) sensor. The PPG sensor can measure the change of the amount of blood in blood vessels of the user by measuring the amount of light that the user's body transmits, using an optical sensor. The PPG sensor may include one or more emitters and one or more receivers. For example, the emitter may include a Light Emitting Diode (LED) and the receiver may include a photodiode (PD). Every time the heart beats, the blood flow in arteries may change while blood vessels expand and contract. That is, in the systolic period of the heart, the amount of blood in blood vessels increases, so the blood vessels expand, and accordingly, the amount of light that is detected by the PD may decrease. In the diastolic period of the heart, the amount of blood in blood vessels decrease and the blood vessels contract, and accordingly, the amount of light that is detected by the PD may increase. It is possible to measure a PPG signal having a cyclic pulse shape showing the change in blood flow by radiating LED light to the tissues of the body of the user and collecting light passing through or reflected using the photodiode. It is possible to determine the number of pulses per second and extract the number of heartbeats, by detecting a Peak-to-Peak Interval (PPI) of the pulse of the PPG signal. Further, it is possible to estimate the Heart Rate Variability (HRV) using a precisely recorded PPI.

However, the disclosure is not limited thereto and the biosensor 620 may include a Laser Diode (LD) or an image sensor.

Unlike the configuration shown in FIG. 6, the various patch or band type sensing device 450 shown in FIG. 3 may replace the function of the sensor module 176 described above. In this case, the sensing device 450 is attached to various portions of the living body and obtains biological information and/or movement information at the various portions. This way, more precise analysis is possible.

The communication module 690 can establish a communication link between the electronic device 101 and an external electronic device (e.g., the server 108 shown in FIG. 1), and can perform communication through the established communication link. For example, the communication module 690 can transmit/receive control commands of data to/from another electronic device, using communication methods such as Bluetooth, BLE, Wi-Fi, NFC, or LTE.

The communication module 690 can transmit information to the external electronic device. For example, the communication module 690 can request an external electronic device having high computing power (e.g., the server 108 shown in FIG. 1) to process data and perform methods described below by transmitting the data measured by the sensor module 176 of the electronic device 200 to the external electronic device.

The communication module 690 can perform communication with the communication module 455 of the sensing device 450 shown in FIG. 3. In this case, the communication module 690 can receive sensing data collected by the sensing device 450 and then transmit the sensing data to the processor 120 or store the sensing data in the memory 130. Alternatively, the communication module 690 may directly transmit the received sensing data to the external electronic device (e.g., the server 108 of FIG. 1) using a communication methods such as Bluetooth, BLE, Wi-Fi, and NFC.

The display 660 can display information. The display 660 may correspond to the display device 160 shown in FIG. 1 or the display 220 shown in FIG. 2C. The display 660 can display measured heartbeat information, stress information and/or a recovery index to the user of the electronic device 101.

The speaker 670 can output sound. The speaker 670 can output a designated audio signal received from the processor 120 (or the audio module 270). The speaker 670 can output notification to the user of the electronic device 101, such as a notification that the recovery index is too low to the user. The notification may include a dialogue or an alarm.

The processor 120 can control the entire operation of the electronic device 101. The processor 120 can receive commands of other components (e.g., the sensor module 176, the communication module 190, and the memory 130), can analyze the received commands, and can perform calculation or process data in accordance with the analyzed commands. The processor 120 may be implemented as hardware such as a chip or a circuit, and may be implemented as a combination of software and hardware. The processor 120 may be a single processor or may be a plurality of processors.

The memory 130 may be one or more memories. The processor 120 can execute commands stored in the memory 130. The memory 130 can store data and/or commands received from or created by other components (e.g., the processor 120, the sensor module 176, the communication module 190, and the display 660).

According to an embodiment, the processor 120 can monitor the activity state of the user using sensing data obtained by the sensor module 176. The processor 120 can monitor whether the activity state of the user who is wearing the electronic device 101 is, for example, an active state, a static state (e.g., static stable state or static unstable state), or a stable state, using the data obtained by the sensor module 176.

According to an embodiment, the processor 120 can store reference patterns for the user's activity state in the memory 130. For example, the memory 130 may contain a reference data pattern for the user's active state. The processor 120 can determine the activity state of the user by comparing the reference patterns for the activity states of the user stored in the memory 130 with at least one signal obtained from the sensor module 176.

According to an embodiment, the processor 120 can periodically obtain sensor values from one or more sensors of the sensor module 176 to monitor the activity state of the user. For example, even though the main processor 121 is inactive (e.g., in the sleep state), the sub-processor 123 can operate with low power and can periodically obtain sensor values. Alternatively, one processor can switch between normal state and low-power state depending on the situation. The processor 120 can track changes in the activity state of the user over time by monitoring the periodically obtained sensor values.

According to an embodiment, the processor 120 can monitor the activity state of the user using the motion sensor 610 and simultaneously can periodically obtain biosignals of the user using the biosensor 620. The processor 120 can extract the HR and/or the HRV by monitoring the heartbeat of the user of the electronic device 101 using the biosensor 620 (e.g., PPG).

According to an embodiment, the processor 120 can predict stress and the sleep pattern of the user of the electronic device 101, using first data obtained from the motion sensor 610 and second data obtained from the biosensor 620.

According to an embodiment, the processor 120 can determine the activity state of the user by comparing motion patterns stored in the memory 130 and the motion pattern of the electronic device 101 measured through the motion sensor 610. For example, when movement of the user is sensed through the motion sensor 610, the processor 120 can determine that the electronic device 101 is in the active state. The active state may be when the user is in motion (e.g., walking or running).

According to an embodiment, the processor 120 may determine the activity state of the user, based on the heart rate obtained using the biosensor 620. For example, if the heart rate of the user has increased in comparison to some baseline, the processor 120 can determine that the user is in an active state. Further, it is also possible to determine whether the user is moving (e.g., walking or running) based on the heart rate.

According to an embodiment, when movement of the electronic device is not sensed by the motion sensor 610, the processor 120 determines that it is in a static state. When the state of biosignals collected from the biosensor 620 are maintained without changing for a predetermined time period, a static stable state is determined. However, when movement of the electronic device is not sensed by the motion sensor 610, but biosignals collected by the biosensor 620 show an increase in heart rate, the processor 120 determines that the user is in a static unstable state.

According to an embodiment, if the user is not in the active or static state, the processor 120 determines that the user is in a stable state. For example, when movement and changes in the biosignal of the user, for example, changes in heart rate and blood pressure, are not sensed for a predetermined time period, the processor 120 can determine that the user is in the stable state.

According to an embodiment, in general, since the reference data of whether the users are in the active state, the static state, or the stable state may be different depending on the biological characteristics of the users, the processor 120 determines reference values for the states of a particular user, based on first data obtained from the motion sensor 610 and second data obtained from the biosensor 620. The processor 120 can determine the intensity of stress of the user by comparing the reference values for the states of the user with the current first data obtained from the motion sensor 610 and/or the current second data obtained from the biosensor 620. As an example, the processor 120 can shows the extent of stress of the user using intensity levels from 1 to 5. As another example, when it is determined that the user is in an unstable state, based on the first data obtained from the motion sensor 610 and the second data obtained from the biosensor 620, and when the heart rate in this case is different from the reference value of the static unstable state, the processor 120 can determine the intensity of stress based on the difference.

In other embodiments, the processor 120 can estimate HR, HRV, pulse and/or blood pressure, based on the second data obtained from the biosensor 620, and can determine stress of the user based on these factors.

According to an embodiment, the processor 120, as shown in FIG. 4 or FIG. 5, can determine the sleep stage of the user based on the HR and/or the HRV. In accordance with an embodiment, the processor 120 can infer the time that the user falls asleep, and the wake-up time, based on the first data obtained from the motion sensor 610. In particular, as for the wake-up time, the processor 120 can determine that the user has woken up and can determine the wake-up time when there were 18 'wake' stages for 20 minutes, where the wake stage is determined based on the HR and/or the HRV. The processor 120 can obtain the HR and/or the HRV using the biosensor 620 while the user sleeps, and can determine whether the sleep state of the user is REM sleep or NREM sleep based on the HR and/or the HRV.

As described above, according to the theory of restoration and recovery through sleep, the REM sleep time may be important for recovery from stress. Accordingly, the processor 120 can estimate the time for which the user was in the REM sleep state from the HR and/or the HRV obtained using the biosensor 620.

According to an embodiment, the processor 120 can calculate a recovery index using the following [Formula 1], based on the time for which REM sleep continued during sleep, the intensity of stress before sleep, and the intensity of stress after sleep.

[Formula 1]

$$\mathrm{a} \times (ST_{REM} + b)^2 + c \times (S_{bS} - S_{aS}) + d = rare\_RQ_{index}$$
$$e * rare\_RQ_{index} + f = RQ_{index}$$

where STREM is the REM sleep time, SbS is the intensity of stress before sleep, SaS is the intensity of stress after sleep, rare_RQindex is a fundamental recovery index value before a recovery index is personalized, and RQindex is a personalized recovery index. Accordingly, parameters for obtaining the recovery index may be discriminated into four generalizing parameters a, b, c, and d that are used to obtain the fundamental recovery index and two personalizing parameters e and f that are used to obtain the personalized index.

According to an embodiment, every day the processor 120 can obtain the time periods of REM sleep during sleep, the intensity of stress before sleep, and the intensity of stress after sleep, and can obtain the generalizing parameters a, b, c, and d in [Formula 1] through testing based on the factors. As an example, it is possible to perform testing on a plurality of users, acquire the time periods of REM sleep, the intensity of stress before sleep, and the intensity of stress after sleep of the test targets, collect the degree of recovery that the test targets feel, and acquire the generalizing parameters a, b, c, and d, based on the collected results. As another example, an external electronic device (e.g., the server 108) that obtain data about a plurality of users can obtain the generalizing parameters and the processor 120 can receive and use the generalizing parameters obtained by the external electronic device.

According to an embodiment, the processor 120 can set the personalizing parameters e and f such that the personalized recovery index (RQindex) is normalized between, for example, 100 and 0. For example, the largest fundamental recover index (rare_RQindex) value obtained in a predetermined number of days (e.g., 30 days) may correspond to the maximum recovery index value (e.g., 100), and the smallest fundamental recovery index value corresponds to the minimum recovery index value (e.g., 0). It is possible to normalize and show the personalized recovery index RQindex as a number within a predetermined range using the personalized parameters. The processor 120 can keep obtaining fundamental recovery index values and can maintain the personalizing parameters e and f values until the fundamental recover index value on a specific day exceeds the largest value used for obtaining the e and f values or until a value smaller than the smallest value is obtained. Alternatively, the processor 120 can maintain the personalizing parameters e and f values until a personalized recovery index value larger than the maximum recovery index value or smaller than the minimum index value is obtained. When the condition is satisfied, the personalizing parameters e and f values can be calculated again. In accordance with an embodiment, the processor 120 can obtain the recovery index on another day after obtaining the personalizing parameters e and f values.

According to an embodiment, the processor 120 can obtain the generalizing parameters a, b, c, and d through testing or experimenting, and can fix and keep using the personalizing parameters e and f after obtaining them once. In another embodiment, the processor 120 can periodically update the generalizing parameters a, b, c, and d and the personalizing parameters e and f. Alternatively, it is possible to periodically change only the personalizing parameters e and f. According to an embodiment, the processor 120 can reset the generalizing parameters a, b, c, and d once a month in response to feedback (questions, etc.) from the user every day for 30 days. In accordance with another embodiment, the processor 120 can reset the generalizing parameters a, b, c, and d using only data for a number of days set in advance in a moving window, or can reset the personalizing parameters e and f while maintaining the generalizing parameters. As an embodiment, the processor 120 can set every day the personalizing parameters e and f such that the largest value of fundamental recovery index values for previous N number of days is the maximum recovery index value and the smallest value is the minimum recovery index value.

According to an embodiment, the processor 120 can provide a separate guide for alleviating stress to the user when the value of the obtained personalized recovery index is smaller than a predetermined value (e.g., 40). As an embodiment, the processor 120 displays that the recovery index is low on the display 660, thereby notifying the user. As another embodiment, the processor 120 can display warning messages such as 'meditate', 'listen to music', or 'get some exercise' on the display 660 to the user when the recovery index is low. In another embodiment, the processor 120 can help the user recover from stress by displaying an application, which can induce corresponding work such as meditation to reduce stress together with the warning messages, on the display 660 or performing the application.

According to an embodiment, an electronic device (e.g., the electronic device 101 shown in FIG. 1 or the electronic device 200 shown in FIG. 2) includes: a communication module configured to perform communication with an external device; a motion sensor configured to sense movement of the electronic device; a biosensor configured to obtain biological information of a user of the electronic device; at least one processor operationally connected with the communication module, the motion sensor, and the biosensor; and at least one memory operationally connected with the at least one processor. The at least one memory stores instructions that, when executed by the at least one processor, cause the at least one processor to: obtain Rapid Eye Movement (REM) sleep time periods and stress data before and after sleep, based on first data obtained by the motion sensor and second data obtained by the biosensor, set parameters for calculating a recovery index, based on the obtained REM sleep time periods and the stress data before and after sleep, and calculate the recovery index, based on the set parameters.

According to an embodiment, the instructions further cause the at least one processor to: determine a falling-asleep time and a wake-up time of the user, based on the first data obtained by the motion sensor, obtain a Heart Rate (HR) and/or a Heart Rate Variation (HRV) of the user, based on the second data obtained by the biosensor, and obtain the REM sleep time periods and/or the stress data before and after sleep, based on the wake-up time and the HR and/or HRV of the user.

According to an embodiment, the instructions further cause the at least one processor to obtain an average value of stress data calculated several times before the falling-asleep time of the user, and obtain an average value of stress data calculated several times after the wake-up time of the user.

According to an embodiment, the parameters for calculating the recovery index include generalizing parameters and personalizing parameters, and the instructions further cause the at least one processor to: set the generalizing parameters, based on the REM sleep time periods and a difference between the stress data before sleep and the stress data after sleep measured for a plurality of users, calculate a fundamental recovery index, based on the set generalizing parameters, and set the personalizing parameters, based on the fundamental recovery index.

According to an embodiment, the instructions further cause the at least one processor to: obtain the fundamental recovery index for a predetermined number of days, and set the personalizing parameters such that a largest value of the fundamental recovery indexes is a maximum fundamental recovery index value and a smallest value of the fundamental recovery indexes is a minimum recovery index value.

According to an embodiment, the instructions further cause the at least one processor to calculate the recovery index, based on the set personalizing parameters and the fundamental recovery index.

According to an embodiment, the instructions further cause the at least one processor to reset the personalizing parameters when the calculated recovery index is larger than the maximum recovery index value or smaller than the minimum recovery index value.

According to an embodiment, the instructions further cause the at least one processor to periodically update the generalizing parameters and the personalizing parameters or periodically update only the personalizing parameters.

According to an embodiment, the instructions further cause the at least one processor to: obtain the fundamental recover index every day for a predetermined number of days, set the personalizing parameters such that a largest value of the fundamental recover indexes is a maximum recovery index value and a smallest value of the fundamental recovery indexes is a minimum recovery index value, and repeatedly calculate the recovery index for the predetermined number of days, based on the set personalizing parameters and the fundamental recovery indexes obtained for the predetermined number of days.

According to an embodiment, the electronic device may further include a display device and the instructions further cause the at least one processor to display the calculated recovery index on the display device.

According to an embodiment, the instructions further cause the at least one processor to display a notification on the display device so that the user recognizes that the recovery index is low, when the calculated recovery index is smaller than a preset value.

According to an embodiment, a system includes a sensing device; and a processing device. The sensing device includes a motion sensor configured to sense movement of a user, a biosensor configured to obtain biological information of the user, and a first communication module configured to perform communication with the processing device. The processing device includes a second communication module configured to perform communication with the sensing device, at least one processor operationally connected with the second communication module, and at least one memory operationally connected with the at least one processor. The sensing device and the processing device are implemented as different pieces of hardware. The sensing device transmits first data obtained by the motion sensor and second data obtained by the biosensor to the processing device through the communication module. The at least one memory of the processing device stores instructions that, when executed by the at least one processor, cause the at least one processor to: obtain Rapid Eye Movement (REM) sleep time periods and stress data before and after sleep, based on first data and second data received from the sensing device, set parameters for calculating a recovery index, based on the obtained REM sleep time periods and/or the stress data before and after sleep, and calculate the recovery index, based on the set parameters.

12

According to an embodiment, the sensing device is a patch type or a band type.

A method of calculating a recovery index in the electronic devices described above and a method of providing a guide for recovery from stress to a user are described hereafter.

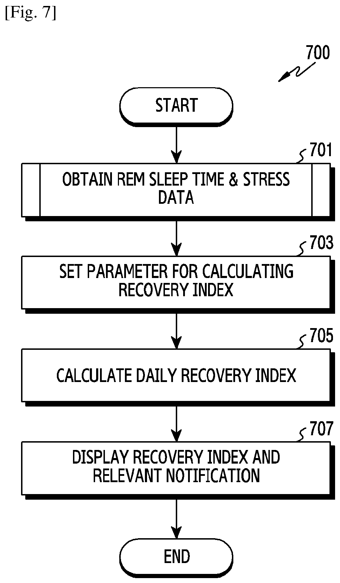

FIG. 7 is a flowchart 700 showing the operation in which an electronic device according to an embodiment calculates a recovery index. The operations of FIG. 7 may be performed by an electronic device (e.g., the electronic device 101 shown in FIG. 1, the server 108 shown in FIG. 1, or the electronic device 200 shown in FIG. 2). Alternatively, the operations of FIG. 7 may be performed by a sensing device (e.g., the sensing device 450 shown in FIG. 3 and a processing device (e.g., the electronic device 101 shown in FIG. 1 or the server 108 shown in FIG. 1)), and the sensing device can measure biological data using a biosensor and can transmit the data through a communication module to a processing device.

According to an embodiment, in operation 701, the electronic devices 101 and 200 can obtain data about REM sleep time, the intensity of stress before sleep, and the intensity of stress after sleep. As an embodiment, the electronic device 101 and 200 can obtain Hear Rate (HR) and Heart Rate Variation (HRV) by monitoring the heartbeat of the user, using the biosensors 620 and 451, and can obtain data about REM sleep time, the intensity of stress before sleep, and the intensity of stress after sleep based on the HR and the HRV.

According to an embodiment, in operation 703, the electronic devices 101 and 200 can set parameters for calculating a recovery index, based on data about an obtained REM sleep time STREM, the intensity of stress SbS before sleep, and the intensity of stress SaS after sleep. For example, the electronic devices 101 and 200 can calculate a personalized recovery index using the [Formula 1], and can use generalizing parameters a, b, c, and d and personalizing parameters e and f in the calculation. The electronic devices 101 and 200 can obtain data about REM sleep time STREM, the intensity of stress SbS before sleep, and the intensity of stress SaS for user's sleep for M number of days (e.g., 30 days), and can reset the generalizing parameters a, b, c, and d and personalizing parameters e and f that are used in the [Formula 1], based on the obtained data for M days (e.g., 30 days).

According to an embodiment, the electronic devices 101 and 200 can fix and keep use the generalizing parameters a, b, c, and d after obtaining them once. In another embodiment, the electronic devices 101 and 200 can periodically change the generalizing parameters a, b, c, and d. In accordance with an embodiment, the electronic devices 101 and 200 can reset the generalizing parameters a, b, c, and d once a month. In accordance with another embodiment, the electronic devices 101 and 200 can reset the generalizing parameters a, b, c, and d using only data for predetermined N days in a moving window. In another embodiment, the electronic devices 101 and 200 can reset the generalizing parameters a, b, c, and d of [Formula 1] through everyday feedback (e.g., questions) from a user. As the generalizing parameters a, b, c, and d are changed, the personalizing parameters e and f can be changed.

According to an embodiment, in operation 705, the electronic devices 101 and 200 can calculate a daily recovery index. In accordance with an embodiment, the electronic devices 101 and 200 can calculate the daily personalized recovery index in accordance with [Formula 1] using the generalizing parameters a, b, c, and d and personalizing parameters e and f set in operation 703. If the parameters a, b, c, d, e, and f are changed, not only is the recovery indexes on the day of the change calculated, but the recovery indices of previous days can also be calculated again using the changed parameters a, b, c, d, e, and f. The electronic devices 101 and 200 can calculate the recovery indexes as relative values.

According to an embodiment, in operation 707, the electronic devices 101 and 200 can display the recovery index and a relevant notification on the display 660. As an embodiment, the electronic devices 101 and 200 can display a graph of recovery indexes obtained for predetermined N days on the display 660. In accordance with another embodiment, the electronic devices 101 and 200 can provide a separate guide for reducing stress to the user when the value of the obtained recovery index is smaller than a predetermined value (e.g., 20). As an embodiment, the electronic devices 101 and 200 display that a recovery index is low on the display 660, thereby notifying the user. As another embodiment, the electronic devices 101 and 200 can display warning messages such as 'take meditation', 'listen to music', or 'take exercise' on the display 660 to the user when the recovery index is low. In another embodiment, the electronic devices 101 and 200 can help the user recover from stress by displaying an application, which can induce corresponding work such as meditation to reduce stress together with the warning messages, on the display 660 or performing the application.

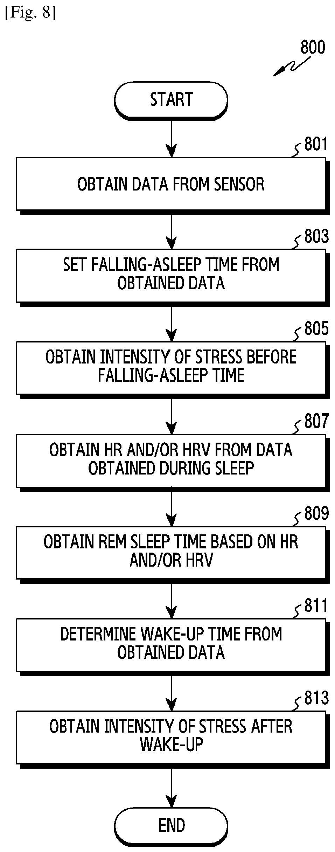

FIG. 8 is a flowchart 800 showing the operation in which an electronic device according to an embodiment obtains REM sleep time and stress data. The flowchart 800 shown in FIG. 8 is an embodiment of operation 701 in FIG. 7, and the operations shown may be understood as being performed by an electronic device (e.g., the electronic device 101 shown in FIG. 1, the server 108 shown in FIG. 1, or the electronic device 200 shown in FIG. 2).

According to an embodiment, in operation 801, the electronic devices 101 and 200 can obtain data from the motion sensors 610 and 453 and/or the biosensors 620 and 451. The motion sensors 610 and 453 can provide first data related to a movement pattern of the electronic device 200 and the biosensors 620 and 451 can obtain second data related to biological information of the user wearing the electronic device 200. In accordance with an embodiment, when first data and/or second data are obtained in a separate electronic device (e.g., the sensing device 450 shown in FIG. 3), the electronic devices 101 and 200 can receive the first data and/or the second data from the separate electronic device using a communication module 690. In accordance with an embodiment, when the flowchart of FIG. 8 is implemented in an external electronic device (e.g., the server 108 shown in FIG. 8), the electronic devices 101 and 200 can transmit the first data and/or the second data to the external electronic device 108 using the communication module 690.

According to an embodiment, in operation 803, the electronic devices 101 and 200 can determine the activity state of the user using the first data obtained from the motion sensors 610 and 453, and can determine the falling-asleep time at which the user falls asleep, based on the determined activity state. As an embodiment, the electronic devices 101 and 200 can determine that the user is asleep when the user is in bed and motion is not sensed for a predetermined time period, based on the first data obtained from the motion sensors 610 and 453. The electronic devices 101 and 200 can additionally determine that the user is asleep when the heart rate is under a predetermined value, or when the change in heart rate is not sensed for a predetermined time period or within a predetermined value, based on the second data obtained from the biosensors 620 and 451.

According to an embodiment, in operation 805, the electronic devices 101 and 200 can obtain the intensity of stress of the user before a falling-asleep time. According to an embodiment, the electronic devices 101 and 200 can obtain first data and second data every 1 minute from the motion sensors 610 and 453 and the biosensors 620 and 451 and can obtain the intensity of stress of the user by comparing the obtained data with reference values stored in the memory. As an embodiment, the electronic devices 101 and 200 can recognize the activity state of the user, based on the first data obtained from the motion sensors 610 and 453, and when the heart rate inferred based on the second data obtained from the biosensor 620 and 451 is different from the reference value in the recognized activity state stored in the memory, the electronic devices 101 and 200 can determine the intensity of stress, based on the difference. According to another embodiment, the electronic devices 101 and 200 can also determine the intensity of stress by averaging five comparison results measured before the falling-asleep time. According to an embodiment, the reference value may depend on users, so the electronic devices 101 and 200 can set reference values for activity states of users through initial setting and then store the reference values in the memory. Actual measurement of the intensity of stress can be performed based on the set reference value.

According to an embodiment, in operation 807, the electronic devices 101 and 200 can obtain HR and/or HRV based on second data obtained from the biosensors 620 and 451 during sleep. The electronic devices 101 and 200 may include a PPG as the biosensors 620 and 451 and can obtain HR and/or HRV based on the PPG. According to an embodiment, the electronic devices 101 and 200 can obtain the HR and/or the HRV every 1 minute.

According to an embodiment, in operation 809, the electronic devices 101 and 200, as shown in FIG. 4 or FIG. 5, can determine the sleep stage of a user, based on the HR and/or the HRV. The electronic devices 101 and 200 can obtain the time periods for which REM sleep occurs.

According to an embodiment, in operation 811, the electronic devices 101 and 200 can determine a wake-up time of a user. In accordance with an embodiment, the electronic devices 101 and 200 can determine that the user has woken up and determine the wake-up time, when the first data obtained from the motion sensors 610 and 453 show that there was movement for over a predetermined time period. In accordance with another embodiment, the electronic devices 101 and 200 can determine that the user has woken up and can determine the wake-up time when there were 18 'wake' stages for 20 minutes, where the wake stage is determined based on the HR and/or the HRV obtained from the biosensors 620 and 451. As another embodiment, when the user provides input showing that the user has woken up to the electronic devices 101 and 200, the electronic devices 101 and 200 determine that the user has woken up and can determined the wake-up time.

According to an embodiment, in operation 813, the electronic devices 101 and 200 can obtain the intensity of stress of the user after the user wakes up. According to an embodiment, the electronic devices 101 and 200 can obtain first data and second data at every 1 minute from the motion sensors 610 and 453 and the biosensors 620 and 451 and can obtain the intensity of stress of the user by comparing the obtained data with reference values stored in the memory. As an embodiment, the electronic devices 101 and 200 can recognize the activity state of the user, based on the first data obtained from the motion sensors 610 and 453, and when the heart rate inferred based on the second data obtained from the biosensor 620 and 451 is different from the reference value in the recognized activity state stored in the memory, the electronic devices 101 and 200 can determine the intensity of stress based on the difference. According to another embodiment, the electronic devices 101 and 200 can also determine the intensity of stress by averaging five comparison results measured after waking-up. According to an embodiment, the reference value may depend on users, so the electronic devices 101 and 200 can set reference values for activity states of users through initial setting and then store the reference values in the memory. Actual measurement of the intensity of stress can be performed based on the set reference value.

Certain embodiments disclosed herein can make the user clearly recognize the sleep quality by displaying the extent of stress recovered through sleep as a recovery index to the user using the method described above.

According to an embodiment, a method of operating an electronic device (e.g., the electronic device 101 shown in FIG.1, the server 108 shown in FIG. 1, and the electronic device 200 shown in FIG. 2) may include: obtaining first data including information about movement of a user; obtaining second data including biological information of the user;

obtaining Rapid Eye Movement (REM) sleep time periods and stress data before and after sleep, based on the first data and the second data; setting parameters for calculating a recovery index, based on the obtained REM sleep time period and stress data before and after sleep; and calculating the recovery index, based on the set parameters.

According to an embodiment, the method further includes determining a falling-asleep time and a wake-up time of the user, based on the first data; obtaining a Heart Rate (HR) and a Heart Rate Variation (HRV) of the user, based on the second data; and obtaining the REM sleep time periods and the stress data before and after sleep, based on the falling-asleep time, the wake-up time, and the HR and/or the HRV of the user.

According to an embodiment, the method further includes obtaining an average value of stress data calculated several times before the falling-asleep time of the user; and obtaining an average value of stress data calculated several times after the wake-up time of the user.

According to an embodiment, the method further includes setting generalizing parameters, based on the REM sleep time periods and a difference between the stress data before sleep and the stress data after sleep measured for a plurality of users; calculating a fundamental recovery index, based on the set generalizing parameters; and setting personalizing parameters, based on the fundamental recovery index.

According to an embodiment, the method further includes obtaining the fundamental recovery index for predetermined number of days; setting the personalizing parameters such that a largest value of the fundamental recovery index values is a maximum recovery index value and a smallest value of the fundamental recovery index values is a minimum recovery index value; and calculating the recovery index, based on the set personalizing parameters and the fundamental recovery index.

According to an embodiment, the method further includes resetting the personalizing parameters when the calculated recovery index is larger than the maximum recovery index value or smaller than the minimum recovery index value.

According to an embodiment, the method further includes displaying the calculated recovery index and a notification on a display device so that the user recognizes that the recovery index is low when the calculated recovery index is smaller than a present value.

An electronic device according to various embodiments disclosed herein may be various types of devices. The electronic devices may include, for example, a portable communication device (e.g., a smart phone), a computer device, a portable multimedia device, a portable medical device, a camera, a wearable device, or a home appliance. According to an embodiment, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," or "connected with,", it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it. This allows the machine to be operated to perform at least one function according to the invoked at least one instruction. At least one instruction may include codes constructed by a compiler or codes that can be executed by an interpreter. A machine-readable storage media may be provided in a non-transitory storage medium type. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., Play Store™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more components of the above-described components or operations may be omitted, or one or more other components or operations may be added. Alternatively or additionally, a plurality of components (e.g., modules or programs) may be integrated into a single component. In this case, integrated components can be performed in the same way as or similarly to performing functions of at least one of components of each of a plurality of components through corresponding components of a plurality of components before integration. According to various embodiments, operations that are performed by a module, a program, or other components may be performed sequentially, in parallel, repeatedly, or heuristically, at least one of the operations may be performed in another order or omitted, or at least one other operation may be added.

Certain of the above-described embodiments of the present disclosure can be implemented in hardware, firmware or via the execution of software or computer code that can be stored in a recording medium such as a CD ROM, a Digital Versatile Disc (DVD), a magnetic tape, a RAM, a floppy disk, a hard disk, or a magneto-optical disk or computer code downloaded over a network originally stored on a remote recording medium or a non-transitory machine readable medium and to be stored on a local recording medium, so that the methods described herein can be rendered via such software that is stored on the recording medium using a general purpose computer, or a special processor or in programmable or dedicated hardware, such as an ASIC or FPGA. As would be understood in the art, the computer, the processor, microprocessor controller or the programmable hardware include memory components, e.g., RAM, ROM, Flash, etc. that may store or receive software or computer code that when accessed and executed by the computer, processor or hardware implement the processing methods described herein.

While the present disclosure has been shown and described with reference to various embodiments thereof, it will be understood by those skilled in the art that various changes in form and details may be made therein without departing from the present disclosure as defined by the appended claims and their equivalents.

## Claims

1. An electronic device comprising:

    a display (160, 220, 660);
    a communication module (190, 455, 690) configured to perform communication with an external device;
    a motion sensor (453, 610) configured to sense movement of the electronic device (101, 200, 300);
    a biosensor (451, 620) configured to obtain biological information of a user of the electronic device;
    at least one processor (120) operationally connected with the communication module (190, 455, 690), the motion sensor (453, 610), and the biosensor (451, 620); and
    at least one memory (130) operationally connected with the at least one processor (120),
    wherein the at least one memory stores instructions that, when executed by the at least one processor, cause the at least one processor to:

        recognize a first activity state of the user, based on motion data obtained from the motion sensor (453, 610),
        when a Heart Rate (HR) obtained from the biosensor (451, 620) is different from a first reference value in the first activity state stored in the at least one memory (130), determine first stress data before sleep based on a difference between the HR and the first reference value,
        recognize a second activity state of the user, based on motion data obtained from the motion sensor (453, 610),
        when a HR obtained from the biosensor (451, 620) is different from a second reference value in the second activity state stored in the at least one memory (130), determine second stress data after sleep based on a difference between the HR and the second reference value,
        obtain Rapid Eye Movement (REM) sleep time periods, based on first data obtained by the motion sensor (453, 610) and second data obtained by the biosensor (451, 620), set parameters for calculating a recovery index, wherein the parameters comprise generalizing parameters for calculating a fundamental recovery index and personalizing parameters for calculating a personalized recovery index,
        calculate the fundamental recovery index, based on the generalizing parameters, the obtained REM sleep time periods and a difference between the first stress data before sleep and the second stress data after sleep,

16

wherein the fundamental recovery index is calculated using following equation:

$$a \times (ST_{REM} + b)^2 + c \times (S_{bS} - S_{aS}) + d = rare\_RQ_{index}$$

wherein a, b, c, and d are the generalizing parameters, $ST_{REM}$ is a total REM sleep time corresponding to the obtained REM sleep time periods, $S_{bS}$ is the first stress data before sleep, $S_{aS}$ is the second stress data after sleep, and $rare\_RQ_{index}$ is the fundamental recovery index,

calculate the personalized recovery index, based on the fundamental recovery index and the personalizing parameters, and

wherein the personalized recovery index is calculated using following equation:

$$e * rare\_RQ_{index} + f = RQ_{index}$$

wherein e and f are the personalizing parameters, $rare\_RQ_{index}$ is the fundamental recovery index, and $RQ_{index}$ is the personalized recovery index,

display, via the display (160, 220, 660), guide information to recover from stress based on the personalized recovery index.

2. The electronic device (101, 200, 300) of claim 1, wherein the instructions further cause the at least one processor to:

determine a falling-asleep time and a wake-up time of the user, based on the first data obtained by the motion sensor (453, 610),
obtain a Heart Rate (HR) and/or a Heart Rate Variation (HRV) of the user, based on the second data obtained by the biosensor (451, 620), and
obtain the REM sleep time periods and/or the stress data before and after sleep, based on the wake-up time and the HR and/or HRV of the user.

3. The electronic device (101, 200, 300) of claim 2, wherein the instructions further cause the at least one processor (120) to obtain an average value of stress data calculated several times before the falling-asleep time of the user, and obtain an average value of stress data calculated several times after the wake-up time of the user.

4. The electronic device (101, 200, 300) of claim 1, wherein the instructions further cause the at least one processor to:

obtain fundamental recovery indexes for a predetermined number of days, and
set personalizing parameters such that a largest value of the fundamental recovery indexes is a maximum fundamental recovery index value and a smallest value of the fundamental recovery indexes is a minimum recovery index value.

5. The electronic device (101, 200, 300) of claim 1, wherein the instructions further cause the at least one processor to:

obtain fundamental recover indexes every day for a predetermined number of days,
set personalizing parameters such that a largest value of the fundamental recover indexes is a maximum recovery index value and a smallest value of the fundamental recovery indexes is a minimum recovery index value, and
repeatedly calculate personalized recovery indexes for the predetermined number of days, based on the set personalizing parameters and the fundamental recovery indexes obtained for the predetermined number of days.

6. The electronic device (101, 200, 300) of claim 1, further comprising a speaker (670), wherein the instructions further cause the at least one processor (120) to output via the speaker (670) output notification to the user of the electronic device

7. The electronic device (101, 200, 300) of claim 1, wherein the instructions further cause the at least one processor (120) to display a notification on the display (160, 220, 660) so that the user recognizes that the personalized recovery index is low, when the personalized recovery index is smaller than a preset value.

8. A method of operating an electronic device (101, 200, 300), the method comprising:

recognizing a first activity state of a user, based on motion data obtained from a motion sensor (453, 610), when a Heart Rate (HR) obtained from a biosensor (451, 620) is different from a first reference value in the first activity state stored in at least one memory (130), determining first stress data before sleep based on a difference between the HR and the first reference value,

recognizing a second activity state of the user, based on motion data obtained from the motion sensor (453, 610), when a HR obtained from the biosensor (451, 620) is different from a second reference value in the second activity state stored in the at least one memory (130), determine second stress data after sleep based on a difference between the HR and the second reference value,

obtain Rapid Eye Movement (REM) sleep time periods, based on first data obtained by the motion sensor (453, 610) and second data obtained by the biosensor (451, 620),

setting parameters for calculating a recovery index, wherein the parameters comprise generalizing parameters for calculating a fundamental recovery index and personalizing parameters for calculating a personalized recovery index,

calculating the fundamental recovery index, based on the generalizing parameters, the obtained REM sleep time periods and a difference between the first stress data before sleep and the second stress data after sleep,

wherein the fundamental recovery index is calculated using following equation:

$$a \times (ST_{REM} + b)^2 + c \times (S_{bS} - S_{aS}) + d = rare\_RQ_{index}$$

wherein a, b, c, and d are the generalizing parameters, $ST_{REM}$ is a total REM sleep time corresponding to the obtained REM sleep time periods, $S_{bS}$ is the first stress data before sleep, $S_{aS}$ is the second stress data after sleep, and rare\_RQ$_{index}$ is the fundamental recovery index,

calculating the personalized recovery index, based on the fundamental recovery index and the personalizing parameters, and

wherein the personalized recovery index is calculated using following equation:

$$e * rare\_RQ_{index} + f = RQ_{index}$$

wherein e and f are the personalizing parameters, rare\_RQ$_{index}$ is the fundamental recovery index, and RQ$_{index}$ is the personalized recovery index,

displaying, via a display (160, 220, 660), guide information to recover from stress based on the personalized recovery index.

9. The method of claim 8, further comprising:

determining a falling-asleep time and a wake-up time of the user, based on the first data;
obtaining a Heart Rate (HR) and a Heart Rate Variation (HRV) of the user, based on the second data; and
obtaining the REM sleep time periods and the stress data before and after sleep, based on the falling-asleep time, the wake-up time, and the HR and/or the HRV of the user.

10. The method of claim 9, further comprising:

obtaining an average value of stress data calculated several times before the falling-asleep time of the user; and
obtaining an average value of stress data calculated several times after the wake-up time of the user.

11. The method of claim 8, further comprising:

obtaining fundamental recovery indexes for predetermined number of days;

setting personalizing parameters such that a largest value of the fundamental recovery index values is a maximum recovery index value and a smallest value of the fundamental recovery index values is a minimum recovery index value; and

calculating the personalized recovery index, based on the set personalizing parameters and the fundamental recovery indexes.

12. The method of claim 11, further comprising resetting the personalizing parameters when the personalized recovery index is larger than the maximum recovery index value or smaller than the minimum recovery index value.

13. The method of claim 8, further comprising:
displaying the personalized recovery index and a notification on a display (160, 220, 660) so that the user recognizes that the personalized recovery index is low when the calculated recovery index is smaller than a present value.

**Patentansprüche**

1. Elektronische Vorrichtung, umfassend:

eine Anzeige (160, 220, 660);
ein Kommunikationsmodul (190, 455, 690), das zum Durchführen der Kommunikation mit einer externen Vorrichtung konfiguriert ist;
einen Bewegungssensor (453, 610), der konfiguriert ist, um die Bewegung der elektronischen Vorrichtung (101, 200, 300) zu erfassen;
einen Biosensor (451, 620), der konfiguriert ist, um biologische Informationen über einen Benutzer der elektronischen Vorrichtung zu erhalten;
mindestens einen Prozessor (120), der betriebsmäßig mit dem Kommunikationsmodul (190, 455, 690), dem Bewegungssensor (453, 610) und dem Biosensor (451, 620) verbunden ist; und
mindestens einen Speicher (130), der mit dem mindestens einen Prozessor (120) betriebsmäßig verbunden ist, wobei der mindestens eine Speicher Instruktionen speichert, die, wenn sie von dem mindestens einen Prozessor ausgeführt werden, den mindestens einen Prozessor veranlassen:

einen ersten Aktivitätszustand des Benutzers auf der Grundlage von vom Bewegungssensor (453, 610) erhaltenen Bewegungsdaten zu erkennen,
wenn eine von dem Biosensor (451, 620) erhaltene Herzfrequenz (HR) von einem ersten Referenzwert in dem ersten Aktivitätszustand, der in dem mindestens einen Speicher (130) gespeichert ist, abweicht, erste Stressdaten vor dem Schlaf auf der Grundlage einer Differenz zwischen der HR und dem ersten Referenzwert zu bestimmen,
einen zweiten Aktivitätszustand des Benutzers auf der Grundlage der vom Bewegungssensor (453, 610) erhaltenen Bewegungsdaten zu erkennen,
wenn eine von dem Biosensor (451, 620) erhaltene HR von einem zweiten Referenzwert in dem zweiten Aktivitätszustand, der in dem mindestens einen Speicher (130) gespeichert ist, abweicht, zweite Stressdaten nach dem Schlaf auf der Grundlage einer Differenz zwischen der HR und dem zweiten Referenzwert zu bestimmen,
Rapid-Eye-Movement-(REM)-Schlafzeiträume auf der Grundlage von ersten Daten, die vom Bewegungssensor (453, 610) erhalten wurden, und zweiten Daten, die vom Biosensor (451, 620) erhalten wurden, zu erhalten,
Parameter zur Berechnung eines Erholungsindexes festzulegen, wobei die Parameter Verallgemeinerungsparameter zur Berechnung eines grundlegenden Erholungsindexes und Personalisierungsparameter zur Berechnung eines personalisierten Erholungsindexes umfassen,
den grundlegenden Erholungsindex auf der Grundlage der Verallgemeinerungsparameter, der erhaltenen REM-Schlaf-Zeiträume und einer Differenz zwischen den ersten Stressdaten vor dem Schlaf und den zweiten Stressdaten nach dem Schlaf zu berechnen,

wobei der grundlegende Erholungsindex unter Verwendung der folgenden Gleichung berechnet wird:

$$a \times (ST_{REM} + b)^2 + c \times (S_{bS} - S_{aS}) + d = rare\_RQ_{index}$$

wobei a, b, c und d die Verallgemeinerungsparameter sind, $ST_{REM}$ eine Gesamt-REM-Schlafzeit ist, die den erhaltenen REM-Schlaf-Zeiträumen entspricht, $S_{bS}$ die ersten Stressdaten vor dem Schlaf ist, $S_{aS}$ die zweiten Stressdaten nach dem Schlaf ist und rare_$RQ_{index}$ der grundlegende Erholungsindex ist,

den personalisierten Erholungsindex auf der Grundlage des grundlegenden Erholungsindexes und der Personalisierungsparameter zu berechnen, und

wobei der personalisierte Erholungsindex unter Verwendung der folgenden Gleichung berechnet wird:

$$e * rare\_RQ_{index} + f = RQ_{index}$$

wobei e und f die Personalisierungsparameter sind, rare_$RQ_{index}$ der grundlegende Erholungsindex und $RQ_{index}$ der personalisierte Erholungsindex ist,

über die Anzeige (160, 220, 660) Leitinformationen zur Erholung von Stress auf der Grundlage des personalisierten Erholungsindexes anzuzeigen.

2. Elektronische Vorrichtung (101, 200, 300) nach Anspruch 1, wobei die Instruktionen ferner den mindestens einen Prozessor zu Folgendem veranlassen:

Bestimmen einer Einschlafzeit und einer Aufwachzeit des Benutzers basierend auf den ersten Daten, die vom Bewegungssensor (453, 610) erhalten wurden,
Erhalten einer Herzfrequenz (HR) und/oder einer Herzfrequenzvariation (HRV) des Benutzers basierend auf den zweiten Daten, die von dem Biosensor (451, 620) erhalten wurden, und
Erhalten der REM-Schlaf-Zeiträume und/oder der Stressdaten vor und nach dem Schlaf auf der Grundlage der Aufwachzeit und der HR und/oder der HRV des Benutzers.

3. Elektronische Vorrichtung (101, 200, 300) nach Anspruch 2, wobei die Instruktionen ferner den mindestens einen Prozessor (120) veranlassen, einen Durchschnittswert von Stressdaten zu erhalten, die mehrere Male vor der Einschlafzeit des Benutzers berechnet wurden, und einen Durchschnittswert von Stressdaten zu erhalten, die mehrere Male nach der Aufwachzeit des Benutzers berechnet wurden.

4. Elektronische Vorrichtung (101, 200, 300) nach Anspruch 1, wobei die Instruktionen ferner den mindestens einen Prozessor veranlassen:

grundlegende Erholungsindizes für eine vorbestimmte Anzahl von Tagen zu erhalten und
die Personalisierungsparameter so festzulegen, dass ein größter Wert der grundlegenden Erholungsindizes ein maximaler grundlegender Erholungsindexwert und ein kleinster Wert der grundlegenden Erholungsindizes ein minimaler Erholungsindexwert ist.

5. Elektronische Vorrichtung (101, 200, 300) nach Anspruch 1, wobei die Instruktionen ferner den mindestens einen Prozessor veranlassen:

jeden Tag für eine vorher festgelegte Anzahl von Tagen grundlegende Erholungsindizes zu erhalten,
Personalisierungsparameter derart festzulegen, dass ein größter Wert der grundlegenden Erholungsindizes ein maximaler Erholungsindexwert ist und ein kleinster Wert der grundlegenden Erholungsindizes ein minimaler Erholungsindexwert ist, und
wiederholt personalisierte Erholungsindizes für die vorbestimmte Anzahl von Tagen basierend auf den festgelegten Personalisierungsparametern und den grundlegenden Erholungsindizes, die für die vorbestimmte Anzahl von Tagen erhalten wurden, zu berechnen.

6. Elektronische Vorrichtung (101, 200, 300) nach Anspruch 1, die ferner einen Lautsprecher (670) umfasst, wobei die Instruktionen ferner den mindestens einen Prozessor (120) veranlassen, über den Lautsprecher (670) eine Ausgabebenachrichtigung an den Benutzer der elektronischen Vorrichtung auszugeben.

7. Elektronische Vorrichtung (101, 200, 300) nach Anspruch 1, wobei die Instruktionen ferner den mindestens einen Prozessor (120) veranlassen, eine Benachrichtigung auf der Anzeige (160, 220, 660) anzuzeigen, so dass der Benutzer erkennt, dass der personalisierte Erholungsindex niedrig ist, wenn der personalisierte Erholungsindex kleiner als ein voreingestellter Wert ist.

8. Verfahren zum Betreiben einer elektronischen Vorrichtung (101, 200, 300), wobei das Verfahren umfasst:

Erkennen eines ersten Aktivitätszustands eines Benutzers auf der Grundlage von Bewegungsdaten, die von einem Bewegungssensor (453, 610) erhalten werden,

wenn eine von einem Biosensor (451, 620) erhaltene Herzfrequenz (HR) von einem ersten Referenzwert in dem ersten Aktivitätszustand, der in mindestens einem Speicher (130) gespeichert ist, abweicht, Bestimmen erster Stressdaten vor dem Schlaf auf der Grundlage einer Differenz zwischen der HR und dem ersten Referenzwert,

Erkennen eines zweiten Aktivitätszustands des Benutzers auf der Grundlage der vom Bewegungssensor (453, 610) erhaltenen Bewegungsdaten,

wenn eine von dem Biosensor (451, 620) erhaltene HR von einem zweiten Referenzwert in dem zweiten Aktivitätszustand, der in dem mindestens einen Speicher (130) gespeichert ist, abweicht, Bestimmen von zweiten Stressdaten nach dem Schlaf auf der Grundlage einer Differenz zwischen der HR und dem zweiten Referenzwert,

Erhalten von Rapid-Eye-Movement-(REM)-Schlafzeiträumen auf der Grundlage von ersten Daten, die vom Bewegungssensor (453, 610) erhalten wurden, und zweiten Daten, die vom Biosensor (451, 620) erhalten wurden,

Festlegen von Parametern zum Berechnen eines Erholungsindexes, wobei die Parameter Verallgemeinerungsparameter zum Berechnen eines grundlegenden Erholungsindexes und Personalisierungsparameter zum Berechnen eines personalisierten Erholungsindexes umfassen,

Berechnen des grundlegenden Erholungsindexes auf der Grundlage der Verallgemeinerungsparameter, der erhaltenen REM-Schlafzeiträume und einer Differenz zwischen den ersten Stressdaten vor dem Schlaf und den zweiten Stressdaten nach dem Schlaf,

wobei der grundlegende Erholungsindex unter Verwendung der folgenden Gleichung berechnet wird:

$$a \times (ST_{REM} + b)^2 + c \times (S_{bS} - S_{aS}) + d = rare\_RQ_{index}$$

wobei a, b, c und d die Verallgemeinerungsparameter sind, $ST_{REM}$ eine Gesamt-REM-Schlafzeit ist, die den erhaltenen REM-Schlafzeiträumen entspricht, $S_{bS}$ die ersten Stressdaten vor dem Schlaf ist, $S_{aS}$ die zweiten Stressdaten nach dem Schlaf ist und rare\_$RQ_{index}$ der grundlegende Erholungsindex ist,

Berechnen des personalisierten Erholungsindexes auf der Grundlage des grundlegenden Erholungsindexes und der Personalisierungsparameter, und

wobei der personalisierte Erholungsindex unter Verwendung der folgenden Gleichung berechnet wird:

$$e * rare\_RQ_{index} + f = RQ_{index}$$

wobei e und f die Personalisierungsparameter sind, rare\_$RQ_{index}$ der grundlegende Erholungsindex und $RQ_{index}$ der personalisierte Erholungsindex ist,

Anzeigen, über eine Anzeige (160, 220, 660), von Leitinformationen zur Erholung von Stress, basierend auf dem personalisierten Erholungsindex.

9. Verfahren nach Anspruch 8, das ferner umfasst:

Bestimmen einer Einschlafzeit und einer Aufwachzeit des Benutzers auf der Grundlage der ersten Daten;
Erhalten einer Herzfrequenz (HR) und einer Herzfrequenzvariation (HRV) des Benutzers basierend auf den zweiten Daten; und
Erhalten der REM-Schlaf-Zeiträume und der Stressdaten vor und nach dem Schlaf basierend auf der Einschlafzeit, der Aufwachzeit und der HR und/oder der HRV des Benutzers.

**10.** Verfahren nach Anspruch 9, das ferner umfasst:

Erhalten eines Durchschnittswertes von Stressdaten, die mehrere Male vor der Einschlafzeit des Benutzers berechnet wurden; und
Erhalten eines Durchschnittswerts der Stressdaten, die mehrere Male nach der Aufwachzeit des Benutzers berechnet wurden.

**11.** Verfahren nach Anspruch 8, das ferner Folgendes umfasst

Erhalten der grundlegenden Erholungsindizes für eine vorbestimmte Anzahl von Tagen;
Festlegen von Personalisierungsparametern derart, dass ein größter Wert der grundlegenden Erholungsindexwerte ein maximaler Erholungsindexwert ist und ein kleinster Wert der grundlegenden Erholungsindexwerte ein minimaler Erholungsindexwert ist; und
Berechnen des personalisierten Erholungsindexes auf der Grundlage der festgelegten Personalisierungsparameter und der grundlegenden Erholungsindizes.

**12.** Verfahren nach Anspruch 11, das ferner das Zurücksetzen der Personalisierungsparameter umfasst, wenn der personalisierte Erholungsindex größer als der maximale Erholungsindexwert oder kleiner als der minimale Erholungsindexwert ist.

**13.** Verfahren nach Anspruch 8, das ferner umfasst:
Anzeigen des personalisierten Erholungsindexes und einer Benachrichtigung auf einer Anzeige (160, 220, 660), so dass der Benutzer erkennt, dass der personalisierte Erholungsindex niedrig ist, wenn der berechnete Erholungsindex kleiner als ein voreingestellter Wert ist.

**Revendications**

**1.** Dispositif électronique comprenant :

un affichage (160, 220, 660) ;
un module de communication (190, 455, 690) configuré pour effectuer la communication avec un dispositif externe ;
un capteur de mouvement (453, 610) configuré pour détecter le mouvement du dispositif électronique (101, 200, 300) ;
un biocapteur (451, 620) configuré pour obtenir des informations biologiques d'un utilisateur du dispositif électronique ;
au moins un processeur (120) connecté de manière opérationnelle au module de communication (190, 455, 690), au capteur de mouvement (453, 610) et au biocapteur (451, 620) ; et
au moins une mémoire (130) connectée de manière opérationnelle à l'au moins un processeur (120),
dans lequel l'au moins une mémoire stocke des instructions qui, lorsqu'elles sont exécutées par l'au moins un processeur, amènent l'au moins un processeur à :

reconnaître un premier état d'activité de l'utilisateur, sur la base de données de mouvement obtenues à partir du capteur de mouvement (453, 610),
lorsqu'une fréquence cardiaque (HR) obtenue à partir du biocapteur (451, 620) est différente d'une première valeur de référence dans le premier état d'activité stocké dans l'au moins une mémoire (130), déterminer des premières données de stress avant le sommeil sur la base d'une différence entre la HR et la première valeur de référence,
reconnaître un deuxième état d'activité de l'utilisateur, sur la base des données de mouvement obtenues à partir du capteur de mouvement (453, 610),
lorsqu'une HR obtenue à partir du biocapteur (451, 620) est différente d'une deuxième valeur de référence dans le deuxième état d'activité stocké dans l'au moins une mémoire (130), déterminer des deuxièmes données de stress après le sommeil sur la base d'une différence entre la HR et la deuxième valeur de référence,
obtenir des périodes de sommeil à mouvements oculaires rapides (REM), sur la base des premières données obtenues par le capteur de mouvement (453, 610) et des deuxièmes données obtenues par le biocapteur (451, 620),

définir des paramètres pour le calcul d'un indice de récupération, les paramètres comprenant des paramètres de généralisation pour le calcul d'un indice de récupération fondamental et des paramètres de personnalisation pour le calcul d'un indice de récupération personnalisé,

calculer l'indice de récupération fondamental, sur la base des paramètres de généralisation, des périodes de sommeil REM obtenues et d'une différence entre les premières données de stress avant le sommeil et les deuxièmes données de stress après le sommeil,

dans lequel l'indice de récupération fondamental est calculé à l'aide de l'équation suivante :

$$a \times (ST_{REM} + b)^2 + c \times (S_{bS} - S_{aS}) + d = rare\_RQ_{index}$$

où a, b, c et d sont les paramètres de généralisation, $ST_{REM}$ est une durée totale de sommeil REM correspondant aux périodes de sommeil REM obtenues, $S_{bS}$ est la première donnée de stress avant le sommeil, $S_{aS}$ est la deuxième donnée de stress après le sommeil, et $rare\_RQ_{index}$ est l'indice de récupération fondamental,

calculer l'indice de récupération personnalisé, sur la base de l'indice de récupération fondamental et des paramètres de personnalisation, et

dans lequel l'indice de récupération personnalisé est calculé à l'aide de l'équation suivante :

$$e * rare\_RQ_{index} + f = RQ_{index}$$

où e et f sont les paramètres de personnalisation, $rare\_RQ_{index}$ est l'indice de récupération fondamental et $RQ_{index}$ est l'indice de récupération personnalisé,

afficher, via l'affichage (160, 220, 660), des informations de guidage pour récupérer du stress en fonction de l'indice de récupération personnalisé.

2. Dispositif électronique (101, 200, 300) de la revendication 1, dans lequel les instructions amènent en outre l'au moins un processeur à :

déterminer une heure d'endormissement et une heure de réveil de l'utilisateur, sur la base des premières données obtenues par le capteur de mouvement (453, 610),
obtenir une fréquence cardiaque (HR) et/ou une variation de fréquence cardiaque (HRV) de l'utilisateur, sur la base des deuxièmes données obtenues par le biocapteur (451, 620), et
obtenir les périodes de sommeil REM et/ou les données de stress avant et après le sommeil, en fonction de l'heure de réveil et de la HR et/ou de la HRV de l'utilisateur.

3. Dispositif électronique (101, 200, 300) de la revendication 2, dans lequel les instructions amènent en outre l'au moins un processeur (120) à obtenir une valeur moyenne des données de stress calculées plusieurs fois avant l'heure d'endormissement de l'utilisateur, et à obtenir une valeur moyenne des données de stress calculées plusieurs fois après l'heure de réveil de l'utilisateur.

4. Dispositif électronique (101, 200, 300) de la revendication 1, dans lequel les instructions amènent en outre l'au moins un processeur à :

obtenir des indices de récupération fondamentaux pour un nombre prédéterminé de jours, et
définir des paramètres de personnalisation de telle sorte que la plus grande valeur des indices de récupération fondamentaux soit une valeur d'indice de récupération fondamental maximale et que la plus petite valeur des indices de récupération fondamentaux soit une valeur d'indice de récupération minimale.

5. Dispositif électronique (101, 200, 300) de la revendication 1, dans lequel les instructions amènent en outre l'au moins un processeur à :

obtenir des indices de récupération fondamentaux tous les jours pendant un nombre de jours prédéterminé,

définir des paramètres de personnalisation de telle sorte que la plus grande valeur des indices de récupération fondamentaux soit une valeur d'indice de récupération maximale et que la plus petite valeur des indices de récupération fondamentaux soit une valeur d'indice de récupération minimale, et

calculer à plusieurs reprises des indices de récupération personnalisés pour le nombre de jours prédéterminé, sur la base des paramètres de personnalisation définis et des indices de récupération fondamentaux obtenus pour le nombre de jours prédéterminé.

**6.** Dispositif électronique (101, 200, 300) de la revendication 1, comprenant en outre un haut-parleur (670),
dans lequel les instructions amènent en outre l'au moins un processeur (120) à émettre via le haut-parleur (670) une notification de sortie à l'intention de l'utilisateur du dispositif électronique.

**7.** Dispositif électronique (101, 200, 300) de la revendication 1, dans lequel les instructions amènent en outre l'au moins un processeur (120) à afficher une notification sur l'affichage (160, 220, 660) afin que l'utilisateur reconnaisse que l'indice de récupération personnalisé est faible, lorsque l'indice de récupération personnalisé est inférieur à une valeur prédéfinie.

**8.** Procédé de fonctionnement d'un dispositif électronique (101, 200, 300), le procédé comprenant :

reconnaître un premier état d'activité d'un utilisateur, sur la base des données de mouvement obtenues à partir d'un capteur de mouvement (453, 610),

lorsqu'une fréquence cardiaque (HR) obtenue à partir d'un biocapteur (451, 620) est différente d'une première valeur de référence dans le premier état d'activité stocké dans au moins une mémoire (130), déterminer des premières données de stress avant le sommeil sur la base d'une différence entre la HR et la première valeur de référence,

reconnaître un deuxième état d'activité de l'utilisateur, sur la base des données de mouvement obtenues à partir du capteur de mouvement (453, 610),

lorsqu'une HR obtenue à partir du biocapteur (451, 620) est différente d'une deuxième valeur de référence dans le deuxième état d'activité stocké dans l'au moins une mémoire (130), déterminer des deuxièmes données de stress après le sommeil sur la base d'une différence entre la HR et la deuxième valeur de référence,

obtenir des périodes de sommeil à mouvements oculaires rapides (REM), sur la base des premières données obtenues par le capteur de mouvement (453, 610) et des deuxièmes données obtenues par le biocapteur (451, 620),

définir des paramètres pour le calcul d'un indice de récupération, les paramètres comprenant des paramètres de généralisation pour le calcul d'un indice de récupération fondamental et des paramètres de personnalisation pour le calcul d'un indice de récupération personnalisé,

calculer l'indice de récupération fondamental, sur la base des paramètres de généralisation, des périodes de sommeil REM obtenues et d'une différence entre les premières données de stress avant le sommeil et les deuxièmes données de stress après le sommeil,

dans lequel l'indice de récupération fondamental est calculé à l'aide de l'équation suivante :

$$a \times (ST_{REM} + b)^2 + c \times (S_{bS} - S_{aS}) + d = rare\_RQ_{index}$$

où a, b, c et d sont les paramètres de généralisation, $ST_{REM}$ est une durée totale de sommeil REM correspondant aux périodes de sommeil REM obtenues, $S_{bS}$ est la première donnée de stress avant le sommeil, $S_{aS}$ est la deuxième donnée de stress après le sommeil, et rare\_$RQ_{index}$ est l'indice de récupération fondamental,

calculer l'indice de récupération personnalisé, sur la base de l'indice de récupération fondamental et des paramètres de personnalisation, et

dans lequel l'indice de récupération personnalisé est calculé à l'aide de l'équation suivante :

$$e * rare\_RQ_{index} + f = RQ_{index}$$

où e et f sont les paramètres de personnalisation, rare\_$RQ_{index}$ est l'indice de récupération fondamental et $RQ_{index}$ est l'indice de récupération personnalisé,

afficher, via un affichage (160, 220, 660), des informations de guidage pour récupérer du stress en fonction de l'indice de récupération personnalisé.

**9.** Procédé de la revendication 8, comprenant en outre :

déterminer une heure d'endormissement et une heure de réveil de l'utilisateur, sur la base des premières données ;
obtenir une fréquence cardiaque (HR) et une variation de fréquence cardiaque (HRV) de l'utilisateur, sur la base des deuxièmes données ; et
obtenir les périodes de sommeil REM et les données de stress avant et après le sommeil, en fonction de l'heure d'endormissement, de l'heure de réveil et de la HR et/ou de la HRV de l'utilisateur.

**10.** Procédé de la revendication 9, comprenant en outre :

obtenir une valeur moyenne des données de stress calculées plusieurs fois avant l'heure d'endormissement de l'utilisateur ; et
obtenir une valeur moyenne des données de stress calculées plusieurs fois après l'heure de réveil de l'utilisateur.

**11.** Procédé de la revendication 8, comprenant en outre :

obtenir des indices de récupération fondamentaux pour un nombre prédéterminé de jours ;
définir des paramètres de personnalisation de telle sorte que la plus grande valeur de l'indice de récupération fondamental soit une valeur d'indice de récupération maximale et que la plus petite valeur de l'indice de récupération fondamental soit une valeur d'indice de récupération minimale ; et
calculer l'indice de récupération personnalisé, sur la base des paramètres de personnalisation définis et des indices de récupération fondamentaux.

**12.** Procédé de la revendication 11, comprenant en outre la réinitialisation des paramètres de personnalisation lorsque l'indice de récupération personnalisé est supérieur à la valeur d'indice de récupération maximale ou inférieur à la valeur d'indice de récupération minimale.

**13.** Procédé de la revendication 8, comprenant en outre :
afficher l'indice de récupération personnalisé et une notification sur un affichage (160, 220, 660) afin que l'utilisateur reconnaisse que l'indice de récupération personnalisé est faible lorsque l'indice de récupération calculé est inférieur à une valeur actuelle.

[Fig. 1]

100

ELECTRONIC DEVICE (101)

| INPUT DEVICE (150) | | MEMORY (130) | PROGRAM (140) |

MEMORY (130)
VOLATILE MEMORY (132)
NON-VOLATILE MEMORY (134)
INTERNAL MEMORY (136)
EXTERNAL MEMORY (138)

PROGRAM (140)
APPLICATION (146)
MIDDLEWARE (144)
OPERATING SYSTEM (142)

INPUT DEVICE (150)
SOUND OUTPUT DEVICE (155)
DISPLAY DEVICE (160)

BATTERY (189)
POWER MANAGEMENT MODULE (188)

PROCESSOR (120)
MAIN PROCESSOR (121)
AUXILIARY PROCESSOR (123)

COMMUNICATION MODULE (190)
WIRELESS COMMUNICATION MODULE (192)
WIRED COMMUNICATION MODULE (194)

SUBSCRIBER IDENTIFICATION MODULE (196)
ANTENNA MODULE (197)

AUDIO MODULE (170)
HAPTIC MODULE (179)
SENSOR MODULE (176)
CAMERA MODULE (180)
INTERFACE (177)
CONNECTING TERMINAL (178)

SECOND NETWORK (199)
ELECTRONIC DEVICE (104)
FIRST NETWORK (198)
ELECTRONIC DEVICE (102)
SERVER (108)

[Fig. 2A]

[Fig. 2B]

[Fig. 2C]

[Fig. 3]

[Fig. 4]

[Fig. 5]

510

501

[Fig. 6]

101

[Fig. 7]

700

START

↓

OBTAIN REM SLEEP TIME & STRESS DATA — 701

↓

SET PARAMETER FOR CALCULATING RECOVERY INDEX — 703

↓

CALCULATE DAILY RECOVERY INDEX — 705

↓

DISPLAY RECOVERY INDEX AND RELEVANT NOTIFICATION — 707

↓

END

[Fig. 8]

800

```
                    ┌──────────┐
                    │  START   │
                    └────┬─────┘
                         │
                         ▼                    ⌇801
        ┌────────────────────────────────────┐
        │      OBTAIN DATA FROM SENSOR        │
        └────────────────┬───────────────────┘
                         │
                         ▼                    ⌇803
        ┌────────────────────────────────────┐
        │     SET FALLING-ASLEEP TIME FROM    │
        │           OBTAINED DATA             │
        └────────────────┬───────────────────┘
                         │
                         ▼                    ⌇805
        ┌────────────────────────────────────┐
        │   OBTAIN INTENSITY OF STRESS BEFORE │
        │         FALLING-ASLEEP TIME         │
        └────────────────┬───────────────────┘
                         │
                         ▼                    ⌇807
        ┌────────────────────────────────────┐
        │    OBTAIN HR AND/OR HRV FROM DATA   │
        │        OBTAINED DURING SLEEP        │
        └────────────────┬───────────────────┘
                         │
                         ▼                    ⌇809
        ┌────────────────────────────────────┐
        │    OBTAIN REM SLEEP TIME BASED ON HR│
        │             AND/OR HRV              │
        └────────────────┬───────────────────┘
                         │
                         ▼                    ⌇811
        ┌────────────────────────────────────┐
        │     DETERMINE WAKE-UP TIME FROM     │
        │           OBTAINED DATA             │
        └────────────────┬───────────────────┘
                         │
                         ▼                    ⌇813
        ┌────────────────────────────────────┐
        │    OBTAIN INTENSITY OF STRESS AFTER │
        │              WAKE-UP                │
        └────────────────┬───────────────────┘
                         │
                         ▼
                    ┌──────────┐
                    │   END    │
                    └──────────┘
```

**EP 3 890 596 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2842491 A1

- US 2015265212 A1